# EUROPEAN PATENT APPLICATION

(11) **EP 4 545 967 A1**
(43) Date of publication of application: **30.04.2025**
(21) Application number: 22967641.6
(22) Date of filing: 09.12.2022
(51) Int. Cl.: G01N 33/543, G01N 33/68

(54) **PREPARATION METHOD FOR IMMUNOMAGNETIC BEAD TEST STRIP FOR RAPIDLY DETECTING TRACE PROTEIN IN CULTURAL RELICS, AND APPLICATION THEREOF**

(30) Priority: 05.12.2022 CN 202211549374
(71) Applicant: China National Silk Museum, Hangzhou, Zhejiang 310002 (CN); Zhejiang Sci-Tech University, Hangzhou, Zhejiang 310000 (CN)
(72) Inventor: ZHENG, Hailing, Hangzhou, Zhejiang 310002 (CN); ZHOU, Yang, Hangzhou, Zhejiang 310002 (CN); YANG, Hailiang, Hangzhou, Zhejiang 310002 (CN); WANG, Bing, Hangzhou, Zhejiang 310018 (CN); PENG, Zhiqin, Hangzhou, Zhejiang 310018 (CN); JIA, Liling, Hangzhou, Zhejiang 310002 (CN)
(74) Representative: Elzaburu S.L.P.
(86) International application number: PCT/CN2022/138004
(87) International publication number: WO 2024/119493

(57) **Abstract**

The present invention relates to the technical field of ancient silk fabric test, and discloses a preparation method and application of immunomagnetic bead test strips for rapid detection of trace proteins in cultural relics. The preparation method includes the following steps: (1) enabling carboxyl magnetic beads and a protein antibody to undergo a coupling reaction, carrying out magnetic separation to remove supernatant, and washing with a buffer and a cleaning agent in sequence to obtain immunomagnetic beads; (2) spraying the immunomagnetic beads onto a pretreated nitrocellulose membrane, and drying for later use; and respectively spraying a diluted antibody solution I and a diluted antibody solution II onto a test line and a control line of the nitrocellulose membrane, and drying for later use; and (3) sequentially assembling a sample pad, an immunomagnetic bead binding pad, the nitrocellulose membrane and an absorbent pad and then cutting the assembled product to obtain immunomagnetic bead test strips, drying, and sealing for storage. The present invention achieves rapid and sensitive detection while enriching trace proteins, thus facilitating the operation at archaeological sites and reducing time consumption.

## Description

### TECHNICAL FIELD

The present invention relates to the technical field of detection of proteins in ancient cultural relics, in particular to a preparation method and application of immunomagnetic bead test strips for rapid detection of trace proteins in cultural relics.

### BACKGROUND

Ancient cultural relics include silk cultural relics and leather cultural relics, the main components of which are proteins. They have been buried in the underground tomb environment for a long time and affected by factors such as humidity, heat and microorganisms, causing varying degrees of degradation of fibers. After the excavation of cultural relics, the drastic changes in environmental conditions further accelerate the aging of the cultural relics. Therefore, in the long course of history, some cultural relics buried in tombs or ruins during that time have long lost their physical appearance, degraded into peptides and amino acids, or become traces, or melted into the soil, and thus are no longer recognizable to the naked eyes. With more and more early ruins being excavated, only the use of modern advanced natural scientific means to extract the information of ancient cultural relics from fragments can provide more archaeological evidence for the study on the origin and dissemination process of silk and leather cultural relics, so that the development of a rapid detection technology for archaeological sites is very critical. However, the amount of proteins remaining in the imprints, residues, and soil is minimal. Therefore, before testing cultural relics, it is necessary to enrich the trace proteins in the cultural relics so as to improve the detection sensitivity, while considering issues including how to simplify the operation and reduce the time consumption. Chinese invention patent with the publication number CN108387435B discloses a method for enriching trace silk fibroin. According to the method, the trace silk fibroin can be enriched through immunomagnetic beads, but after enrichment, it needs to be eluted and then detected by ELISA. The process is relatively complicated and takes a long time, thus being not suitable for rapid detection at archaeological sites. Chinese invention patent with the publication number CN104459162B discloses a preparation method of indirect competition method test paper for ancient silk fabrics, and Chinese invention patent with the publication number CN104459118B discloses a preparation method of double-antibody sandwich method test paper for ancient silk fabrics. Although the test paper can be used for rapid detection of silk fabric residues at archaeological sites, a more sensitive detection method is required for detecting residues in small amounts at the archaeological sites.

### SUMMARY

In order to solve the technical problem of how to quickly detect trace proteins at archaeological sites, the present invention provides a preparation method and application of immunomagnetic bead test strips for rapid detection of trace proteins in cultural relics. By combining magnetic beads with an immunochromatography technology, trace proteins can be quickly detected at archaeological sites with convenient operation and less time consumption.

The specific technical solutions of the present invention are:
In the first aspect, the present invention provides a preparation method of immunomagnetic bead test strips for rapid detection of trace proteins in cultural relics, which includes the following steps:
(1) enabling carboxyl magnetic beads and a protein antibody to undergo a coupling reaction, carrying out magnetic separation to remove supernatant, and washing with a buffer and a cleaning agent in sequence to obtain immunomagnetic beads;
(2) spraying the immunomagnetic beads onto a pretreated nitrocellulose membrane, and drying for later use; and respectively spraying a diluted antibody solution I and a diluted antibody solution II onto a test line and a control line of the nitrocellulose membrane, and drying for later use; and
(3) sequentially assembling a sample pad, an immunomagnetic bead binding pad, the nitrocellulose membrane and an absorbent pad and then cutting the assembled product to obtain immunomagnetic bead test strips, drying, and sealing for storage.

By combining the magnetic beads with immunochromatography test paper, the present invention can directly test whether a sample to be tested contains protein on the test strip while rapidly enriching the protein in the sample to be tested, which avoids the elution step in the prior art, and improves the detection sensitivity of the existing test paper, so that the operation is simple, and the rapid detection at archaeological sites is facilitated.

Preferably, when the trace protein is silk fibroin, the protein antibody is a goat anti-rabbit silk fibroin polyclonal antibody; the antibody solution I is a mouse anti-rabbit silk fibroin polyclonal antibody solution; and the antibody solution II is a goat anti-rabbit antibody solution. The goat anti-rabbit silk fibroin polyclonal antibody uses a silk protein hydrolyzate as antigen, which can stimulate an immune body to produce antibodies with different binding sites. Due to the continuity of silk fibroin molecules, the continuity of the silk fibroin antibody can be guaranteed, and it can also ensure that the silk fibroin antibody also has many sites that can bind to the silk fibroin. The immunomagnetic beads prepared by using the silk fibroin antibody also have more sites that can bind to the silk fibroin, ensuring the enrichment effect of the magnetic beads. The test line antibody is the mouse anti-rabbit silk fibroin polyclonal antibody, which can effectively capture the silk fibroin. Through the double-antibody sandwich method, the test line antibody can also capture the immunomagnetic beads coupled to the silk fibroin antibody. The control line antibody is the goat anti-rabbit antibody, which only captures the antibody coupled to the magnetic beads. As long as the test strip can be used, the control line will undergo a chromogenic reaction under any circumstances.

When the trace protein is collagen, the protein antibody is a goat anti-rabbit type I collagen polyclonal antibody; the antibody solution I is a mouse anti-rabbit type I collagen polyclonal antibody solution; and the antibody solution II is a goat anti-rabbit antibody solution.

Preferably, in step (1), the coupling reaction includes the following steps: adding the carboxyl magnetic beads into an MES buffer for resuspension, then adding the protein antibody and a coupling activator, and performing the coupling reaction at room temperature for 2-4 h.

Preferably, in step (1), the sizes of the carboxyl magnetic beads are 100-300 nm; the mass ratio of the carboxyl magnetic beads to the protein antibody is 10 mg:500 µg; the pH of the MES buffer is 5-7; and the coupling activator is an EDC·HCl solution with a concentration of 10 mg/mL.

The most suitable sizes of the carboxyl magnetic beads are 100-300 nm. If the sizes are too small, the chromatography on the nitrocellulose membrane will be too fast, resulting in false positives. If the sizes are too large, there will be failure in chromatography on the membrane. MES buffers with different pH values will cause different coupling rates of the magnetic beads, and the magnetic beads with the sizes in the range have the best coupling rates.

Preferably, in step (1), the buffer is a Tris-HCl solution, with a pH value being 7.4; and the cleaning agent is a PBST solution containing 0.1 percent by mass of Tween-20, with a pH value being 7.4.

Preferably, in step (1), the step of washing with a buffer and a cleaning agent in sequence includes: adding the carboxyl magnetic beads into the buffer, mixing and resuspending the magnetic beads, rotating and mixing for 2-3 h at room temperature, carrying out magnetic separation to remove supernatant, then adding the cleaning agent, and carrying out magnetic separation to remove supernatant.

Preferably, in step (2), the pretreatment refers to soaking in the MES buffer and then drying; and the dilution refers to diluting 500-1000 times with a PBS buffer containing 0.5-2.0 percent by mass of sucrose.

When the pure PBS buffer is used as a test paper antibody diluent, the immunomagnetic beads may not perform chromatography properly in the chromatography process. Adding sucrose can reduce the diffusion rate of the antibody solution, allowing the nitrocellulose membrane to be fully infiltrated and ensuring the smooth chromatography of the immunomagnetic beads.

Preferably, in step (1), a preparation method of the carboxyl magnetic beads includes the following steps:
(a) preparation of SiO₂@Fe₃O₄ magnetic microspheres: adding ferric acetylacetonate and 1,12-dodecanediol into phenyl ether for dissolving, and enabling same to react at 180-210°C for 5-20 min; and adding an ethanol aqueous solution and mixing, then adding ethyl orthosilicate dropwise, heating up to 80-100°C to perform a reaction for 6-10 h, washing and drying at the end of the reaction, so as to obtain SiO₂@Fe₃O₄ magnetic microspheres;
(b) preparation of octaglycidyl-polyhedral oligomeric silsesquioxane: mixing octavinyl-polyhedral oligomeric silsesquioxane, concentrated sulfuric acid, platinum-1,3-divinyl-1,1,3,3-tetramethyldisiloxane and toluene, heating and refluxing under an inert atmosphere, adding allyl glycidyl ether and trichloromethane dropwise, and continuing heating and refluxing for 8-12 h; and after the reaction is completed, distilling under reduced pressure, followed by drying, so as to obtain octaglycidyl-polyhedral oligomeric silsesquioxane; and
(c) preparation of carboxyl magnetic beads: dispersing SiO₂@Fe₃O₄ magnetic microspheres in a mixed solution of octaglycidyl-polyhedral oligomeric silsesquioxane, a silane coupling agent and water, stirring for a reflux reaction for 2-4 h, then carrying out magnetic attraction, and washing; dispersing the resultant in a Tris-HCl buffer with a pH of 8-9, adding dopamine and stannous octoate, and enabling same to be subjected to a constant temperature water bath reaction at 30-40°C for 6-8 h; and filtering by magnetic attraction, then adding carboxymethyl chitosan, glutaraldehyde, glacial acetic acid and water and mixing, and enabling the obtained mixture to react at 60-70°C for 3-5 h while stirring, followed by magnetic attraction, washing and drying, so as to obtain carboxyl magnetic beads.

The iron salt will decompose to generate Fe₃O₄ magnetic nanoparticles, and then the surfaces of the magnetic nanoparticles are coated with ethyl orthosilicate to form core-shell structured SiO₂@Fe₃O₄ magnetic microspheres. These microspheres are sequentially bonded with epoxy groups and carboxyl groups in a layered manner to increase the density of surface functional groups, so that the antibody coupling rates of the carboxyl magnetic beads are ultimately improved. The octaglycidyl-polyhedral oligomeric silsesquioxane has a cage type structure whose inner core is composed of silicon-oxygen-silicon bonds (Si-O-Si) and outer shell is formed by epoxy groups. It has good affinity with the SiO₂ layers on the surfaces of magnetic microspheres. Moreover, the carboxyl magnetic beads, obtained by grafting and branching on the epoxy groups and distributed outside the cage type structure, have better distribution uniformity of carboxyl groups, which can improve the effective availability of the carboxyl magnetic beads and the enrichment effect of silk fibroin. Dopamine can not only undergo a grafting reaction with epoxy groups, but also self-polymerize to form a coated layer structure, which can enable the residual hydroxyl and amino groups in the outer layer to react with the added carboxymethyl chitosan by controlling the degree of reaction while improving the structural stability, thus successfully introducing reactive carboxyl groups. The carboxyl magnetic beads obtained by layer-by-layer coating and cross-linking are better in coating properties, better in distribution uniformity of carboxyl groups on the surfaces, and more uniform in particle size distribution of magnetic beads, which is conducive to the effective coupling of antibodies.

Preferably, in step (a), the molar ratio of the ferric acetylacetonate to the ethyl orthosilicate is (5-10):(2-4).

Preferably, in step (b), the amount ratio of the octavinyl-polyhedral oligomeric silsesquioxane, the concentrated sulfuric acid, the platinum-1,3-divinyl-1,1,3,3-tetramethyldisiloxane, and the toluene is (10-20 g):(5-10 mL):(1-3 g):100 mL; and the amount ratio of the octavinyl-polyhedral oligomeric silsesquioxane, the allyl glycidyl ether, and the trichloromethane is (7-12 g):(11-15 g):(20-40 mL).

The use of allyl glycidyl ether for grafting epoxy groups can provide polyhedral oligomeric silsesquioxane with flexible alkyl carbon chain segments, and extend the length of spacer arms between the magnetic beads and surface functional groups, which can achieve more effective coupling.

Preferably, in step (c), the mass ratio of the SiO₂@Fe₃O₄ magnetic microspheres, the octaglycidyl-polyhedral oligomeric silsesquioxane, and the dopamine is 1:(2-3):(2-5); the mass ratio of the dopamine, the carboxymethyl chitosan, and the glutaraldehyde is (2-5):(1-2):(5-10); and the carboxymethyl substitution degree of the carboxymethyl chitosan is not greater than 1. Because dopamine will also undergo self-polymerization, controlling the mass ratio of raw materials can ensure that sufficient cross-linked amino groups and hydroxyl groups remain to improve the binding stability. In addition, since the carboxymethyl substitution degree of carboxymethyl chitosan is not greater than 1, reactive functional groups can effectively bind chitosan to magnetic beads while ensuring a sufficiently high surface carboxyl group density.

In the second aspect, the present invention also provides application of the immunomagnetic bead test strips prepared by the above preparation method, including the following steps: dissolving a sample to be tested in a protein solubilization reagent, taking supernatant and dripping same onto a sample pad of the immunomagnetic bead test strip; after chromatography, observing the color development in a test line and a control line on the immunomagnetic bead test strip; and under the premise of the color development in the control line, if there is no color in the test line, it represents negative, and if there is color in the test line, it represents positive. The contents of protein degradation products remaining in the imprints, residues and soil are minimal, and the molecular weights thereof are low, so that the protein degradation products can be dissolved and extracted under mild conditions. After being extracted by PBS, the solution can be directly tested on the test paper without further treatment, which is convenient for the detection at archaeological sites.

Preferably, when the trace protein is silk fibroin, the silk fibroin solubilization reagent is a carbonate buffer solution with a pH of 9.6 or a CaCl₂ solution with a mass fraction of 50%, where the dissolution temperature is 60-80°C, and the dissolution time is 45-90 min.

Compared with the prior art, the present invention has the following beneficial effects:
(1) the present invention utilizes the protein antibody to prepare the immunomagnetic beads, and combines the immunomagnetic beads with a chromatography technology to prepare immunomagnetic bead chromatography test paper, which realizes rapid and sensitive detection while enriching trace proteins;
(2) the carboxyl magnetic beads obtained by layer-by-layer coating and cross-linking are better in coating properties, better in distribution uniformity of carboxyl groups on the surfaces, and more uniform in particle size distribution of magnetic beads, which is conducive to achieving the effective coupling of antibodies and improving the antibody coupling rates; and
(3) after cultural relic samples are dissolved, they can be directly tested on the immunomagnetic bead test strips without further treatment, thus facilitating the operation at archaeological sites and reducing time consumption.

### BRIEF DESCRIPTION OF THE DRAWINGS

FIG. 1 shows the sensitivity test results of colloidal gold immunochromatography test paper in Embodiment 1 (the upper and lower lines are a C line and a T line, respectively);
FIG. 2 shows the sensitivity test results of the colloidal gold immunochromatography test paper after the enrichment with immunomagnetic beads in Embodiment 1;
FIG. 3 shows the test result of a mineralized cultural relic sample of textiles in Embodiment 1; and
FIG. 4 shows the affected coupling rate results in Embodiment 4 and Embodiment 5.

### DETAILED DESCRIPTION

The present invention will be further described with reference to the embodiments below.

### Embodiment 1

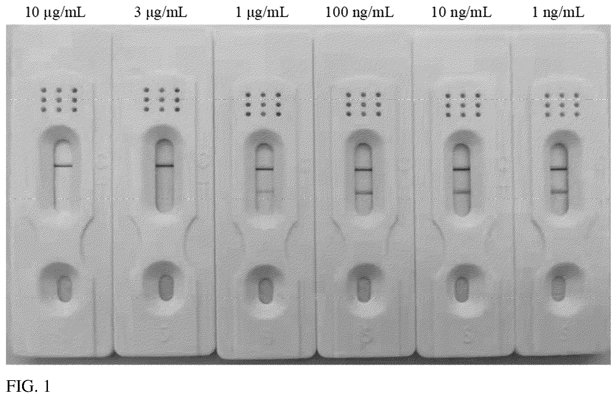

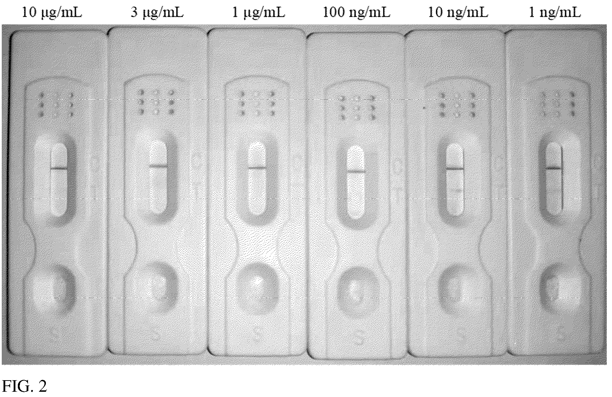

A preparation method of immunomagnetic bead test strips for rapid detection of trace silk fibroin includes the following steps:
(1) Obtaining of immunomagnetic beads: 10 mg of 1 mL 100 nm carboxyl magnetic beads were added into a 2 mL centrifuge tube, and magnetic separation was carried out to remove supernatant; 400 µL of a 100 mmol/L MES buffer with a pH of 5.0 was added for resuspending the magnetic beads; and 500 µg of a goat anti-rabbit silk fibroin polyclonal antibody was added, 100 µL of a 10 mg/mL coupling activator EDC·HCl solution was then added, and the obtained mixture was placed on an oscillator at room temperature for a coupling reaction for 2 h. After the reaction was completed, the products were collected by a hopper magnet, and the supernatant was removed; 1 mL of a Tris-HCl buffer with a pH of 7.4 was added and mixed for resuspending the magnetic beads, the obtained mixture was rotated and mixed at room temperature for 2 h, and magnetic separation was carried out to remove supernatant; and 1 mL of a PBST (containing 0.1% of Tween-20) cleaning agent with a pH of 7.4 was added, and magnetic separation was carried out to remove supernatant, so that immunomagnetic beads were obtained.
(2) Enrichment effect evaluation: PBS 7.4 was used to prepare silk fibroin standard solutions with concentrations of 1 ng/mL, 10 ng/mL, 100 ng/mL, 1.0 µg/mL, 3.0 µg/mL and 10 µg/mL. As shown in FIG. 1, 500 µL of the silk fibroin standard solutions were respectively taken and directly tested by test paper developed by patent CN104459162B. In addition, 500 µL of the silk fibroin standard solutions were respectively taken and dissolved in centrifuge tubes, immunomagnetic beads were added for enrichment, the solution was removed after 30 min, and the products were eluted with methanol. As shown in FIG. 2, after drying and redissolving, the enrichment effect of the immunomagnetic beads was evaluated using the test paper developed by patent CN104459162B.
(3) Preparation of immunomagnetic bead test strips: a nitrocellulose membrane (UniSart CN140) was soaked in an MES buffer with a pH of 5.0 for 30 min, and then dried in an oven at 37°C. The prepared immunomagnetic beads were evenly sprayed onto the pretreated nitrocellulose membrane with an airjet spray nozzle of a membrane spraying instrument, and dried at 37°C for later use. A mouse anti-rabbit silk fibroin polyclonal antibody solution and a goat anti-rabbit antibody solution diluted 500 times in 20 µL of a PBS buffer containing 0.5 percent by mass of sucrose were respectively sprayed onto a test line (T) and a control line (C) of the nitrocellulose membrane with a length of 2.5 cm and a width of 4 mm using a membrane sprayer, the distance between the test line and the control line was ensured to be 10 mm, and the product was dried at 37°C for later use. A sample pad, an immunomagnetic bead binding pad, the nitrocellulose membrane and an absorbent pad were sequentially assembled on a PVC substrate, and then cut into 4 mm-wide independent test strips with a membrane cutter, and a desiccant was put into the test strips for sealed storage.

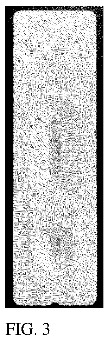

The immunomagnetic bead test strip prepared above was used for sample test:
1 mg of a mineralized sample of textiles was taken and dissolved in 100 µL of a carbonate buffer solution with a pH of 9.6 at 80°C, and then stirred evenly; after 90 min, three drops of supernatant were taken and dripped onto a sample pad of the immunomagnetic bead test strip; and after 10 min of chromatography, the color development in the T line and C line of the test strip was observed. The final identification result of the textile cultural relics in Embodiment 1 is shown in FIG. 3, where both the C and T lines are colored, indicating that the fiber material of the mineralized sample is silk.

### Embodiment 2

A preparation method of immunomagnetic bead test strips for rapid detection of trace silk fibroin includes the following steps:
(1) Obtaining of immunomagnetic beads: 10 mg of 1 mL 200 nm carboxyl magnetic beads were added into a 2 mL centrifuge tube, and magnetic separation was carried out to remove supernatant; 400 µL of a 100 mmol/L MES buffer with a pH of 6.0 was added for resuspending the magnetic beads; and 500 µg of a goat anti-rabbit silk fibroin polyclonal antibody was added, 100 µL of a 10 mg/mL coupling activator EDC·HCl solution was then added, and the obtained mixture was placed on an oscillator at room temperature for a coupling reaction for 2 h. After the reaction was completed, the products were collected by a hopper magnet, and the supernatant was removed; 1 mL of a Tris-HCl buffer with a pH of 7.4 was added and mixed for resuspending the magnetic beads, the obtained mixture was rotated and mixed at room temperature for 2 h, and magnetic separation was carried out to remove supernatant; and 1 mL of a PBST (containing 0.1% of Tween-20) cleaning agent with a pH of 7.4 was added, and magnetic separation was carried out to remove supernatant, so that immunomagnetic beads were obtained.
(2) Preparation of immunomagnetic bead test strips: a nitrocellulose membrane (UniSart CN140) was soaked in an MES buffer with a pH of 5.0 for 30 min, and then dried in an oven at 37°C. The prepared immunomagnetic beads were evenly sprayed onto the pretreated nitrocellulose membrane with an airjet spray nozzle of a membrane spraying instrument, and dried at 37°C for later use. A mouse anti-rabbit silk fibroin polyclonal antibody solution and a goat anti-rabbit antibody solution diluted 500 times in 20 µL of a PBS buffer containing 1.0 percent by mass of sucrose were respectively sprayed onto a test line (T) and a control line (C) of the nitrocellulose membrane with a length of 2.5 cm and a width of 4 mm using a membrane sprayer, the distance between the test line and the control line was ensured to be 10 mm, and the product was dried at 37°C for later use. A sample pad, an immunomagnetic bead binding pad, the nitrocellulose membrane and an absorbent pad were sequentially assembled on a PVC substrate, and then cut into 4 mm-wide independent test strips with a membrane cutter, and a desiccant was put into the test strips for sealed storage.

The immunomagnetic bead test strip prepared above was used for sample test:
2 mg of an imprint sample of textiles was taken and dissolved in 1 mL of a carbonate buffer solution with a pH of 9.6 at 70°C, and then stirred evenly; after 60 min, three drops of supernatant were taken and dripped onto a sample pad of the immunomagnetic bead test strip; and after 10 min of chromatography, the color development in the T line and C line of the test strip was observed. The final identification result of the textile cultural relics in Embodiment 1 is that both the C and T lines are colored, indicating that the fiber material of the imprint sample is silk.

### Embodiment 3

A preparation method of immunomagnetic bead test strips for rapid detection of trace silk fibroin includes the following steps:
(1) Obtaining of immunomagnetic beads: 10 mg of 1 mL 300 nm carboxyl magnetic beads were added into a 2 mL centrifuge tube, and magnetic separation was carried out to remove supernatant; 400 µL of a 100 mmol/L MES buffer with a pH of 7.0 was added for resuspending the magnetic beads; and 500 µg of a goat anti-rabbit silk fibroin polyclonal antibody was added, 100 µL of a 10 mg/mL coupling activator EDC·HCl solution was then added, and the obtained mixture was placed on an oscillator at room temperature for a coupling reaction for 2 h. After the reaction was completed, the products were collected by a hopper magnet, and the supernatant was removed; 1 mL of a Tris-HCl buffer with a pH of 7.4 was added and mixed for resuspending the magnetic beads, the obtained mixture was rotated and mixed at room temperature for 2 h, and magnetic separation was carried out to remove supernatant; and 1 mL of a PBST (containing 0.1% of Tween-20) cleaning agent with a pH of 7.4 was added, and magnetic separation was carried out to remove supernatant, so that immunomagnetic beads were obtained.
(2) Preparation of immunomagnetic bead test strips: a nitrocellulose membrane (UniSart CN140) was soaked in an MES buffer with a pH of 5.0 for 30 min, and then dried in an oven at 37°C. The prepared immunomagnetic beads were evenly sprayed onto the pretreated nitrocellulose membrane with an airjet spray nozzle of a membrane spraying instrument, and dried at 37°C for later use. A mouse anti-rabbit silk fibroin polyclonal antibody solution and a goat anti-rabbit antibody solution diluted 1000 times in 20 µL of a PBS buffer containing 1.5 percent by mass of sucrose were respectively sprayed onto a test line (T) and a control line (C) of the nitrocellulose membrane with a length of 2.5 cm and a width of 4 mm using a membrane sprayer, the distance between the test line and the control line was ensured to be 10 mm, and the product was dried at 37°C for later use. A sample pad, an immunomagnetic bead binding pad, the nitrocellulose membrane and an absorbent pad were sequentially assembled on a PVC substrate, and then cut into 4 mm-wide independent test strips with a membrane cutter, and a desiccant was put into the test strips for sealed storage.

The immunomagnetic bead test strip prepared above was used for sample test:
10 mg of a soil sample was taken and dissolved in 1 mL of a carbonate buffer solution with a pH of 9.6 at 70°C, and then stirred evenly; after 45 min, three drops of supernatant were taken and dripped onto a sample pad of the immunomagnetic bead test strip; and after 10 min of chromatography, the color development in the T line and C line of the test strip was observed. The final identification result of the textile cultural relics in Embodiment 1 is that both the C and T lines are colored, indicating that the soil sample contains silk.

### Embodiment 4

Testing the effect of different levels of goat anti-rabbit silk fibroin polyclonal antibodies on the coupling rates of carboxyl magnetic beads:
10 mg of 1 mL 100 nm carboxyl magnetic beads were added into a 2 mL centrifuge tube, and magnetic separation was carried out to remove supernatant; 400 µL of a 100 mmol/L MES buffer with a pH of 5.0 was added for resuspending the magnetic beads; different levels of goat anti-rabbit silk fibroin polyclonal antibodies were separately added, where 8 parallel groups of the goat anti-rabbit silk fibroin polyclonal antibodies were set up according to their different levels, and the antibody contents (calculated by dividing the amounts of antibodies added by the amounts of carboxyl magnetic beads added) were 30 µg/mg, 40 µg/mg, 50 µg/mg, 60 µg/mg, 70 µg/mg, 80 µg/mg, 90 µg/mg, and 100 µg/mg, respectively; and 100 µL of a 10 mg/mL coupling activator EDC·HCl solution was then added, and the obtained mixture was placed on an oscillator at room temperature for a coupling reaction for 2 h. After the reaction was completed, the carboxyl magnetic beads were collected by a hopper magnet, and the coupling rates were calculated.

### Embodiment 5

Testing the effect of MES buffers with different pH values on the coupling rates of carboxyl magnetic beads:
10 mg of 1 mL 100 nm carboxyl magnetic beads were added into a 2 mL centrifuge tube, and magnetic separation was carried out to remove supernatant; 400 µL of 100 mmol/L MES buffers were separately added for resuspending the magnetic beads, where 5 parallel groups of the MES buffers were set up according to their different pH values, and the pH values were 5.0, 5.5, 6.0, 6.5, and 7.0, respectively; and 500 µg of a goat anti-rabbit silk fibroin polyclonal antibody was added, 100 µL of a 10 mg/mL coupling activator EDC·HCl solution was then added, and the obtained mixture was placed on an oscillator at room temperature for a coupling reaction for 2 h. After the reaction was completed, the carboxyl magnetic beads were collected by a hopper magnet, and the coupling rates were calculated.

### Embodiment 6

A preparation method of immunomagnetic bead test strips for rapid detection of trace collagen includes the following steps:
(1) Obtaining of immunomagnetic beads: 10 mg of 1 mL 100 nm carboxyl magnetic beads were added into a 2 mL centrifuge tube, and magnetic separation was carried out to remove supernatant; 400 µL of a 100 mmol/L MES buffer with a pH of 5.0 was added for resuspending the magnetic beads; and 500 µg of a goat anti-rabbit type I collagen polyclonal antibody was added, 100 µL of a 10 mg/mL coupling activator EDC·HCl solution was then added, and the obtained mixture was placed on an oscillator at room temperature for a coupling reaction for 3 h. After the reaction was completed, the products were collected by a hopper magnet, and the supernatant was removed; 1 mL of a Tris-HCl buffer with a pH of 7.4 was added and mixed for resuspending the magnetic beads, the obtained mixture was rotated and mixed at room temperature for 4 h, and magnetic separation was carried out to remove supernatant; and 1 mL of a PBST (containing 0.1% of Tween-20) cleaning agent with a pH of 7.4 was added, and magnetic separation was carried out to remove supernatant, so that immunomagnetic beads were obtained.
(2) Preparation of immunomagnetic bead test strips: a nitrocellulose membrane (UniSart CN140) was soaked in an MES buffer with a pH of 5.0 for 30 min, and then dried in an oven at 37°C. The prepared immunomagnetic beads were evenly sprayed onto the pretreated nitrocellulose membrane with an airjet spray nozzle of a membrane spraying instrument, and dried at 37°C for later use. A mouse anti-rabbit type I collagen polyclonal antibody solution and a goat anti-rabbit antibody solution diluted 500 times in 20 µL of a PBS buffer containing 0.5 percent by mass of sucrose were respectively sprayed onto a test line (T) and a control line (C) of the nitrocellulose membrane with a length of 2.5 cm and a width of 4 mm using a membrane sprayer, the distance between the test line and the control line was ensured to be 10 mm, and the product was dried at 37°C for later use. A sample pad, an immunomagnetic bead binding pad, the nitrocellulose membrane and an absorbent pad were sequentially assembled on a PVC substrate, and then cut into 4 mm-wide independent test strips with a membrane cutter, and a desiccant was put into the test strips for sealed storage.

The immunomagnetic bead test strip prepared above was used for sample test:
1 mg of a fiber residue sample from an archaeological site was taken and dissolved in 100 µL of a carbonate buffer solution with a pH of 9.6 at 80°C, and then stirred evenly; after 120 min, three drops of supernatant were taken and dripped onto a sample pad of the immunomagnetic bead test strip; and after 20 min of chromatography, the color development in the T line and C line of the test strip was observed. Both the C and T lines are colored, indicating that the fiber residue sample contains collagen, and the fibers are leather collagen fibers.

### Embodiment 7

A preparation method of carboxyl magnetic beads includes the following steps:
(a) preparation of SiO₂@Fe₃O₄ magnetic microspheres: 2.47 g of ferric acetylacetonate (353.17) and 7.08 g of 1,12-dodecanediol (202.33, 5) were added into 50 mL of phenyl ether for dissolving, and enabled to react at 190°C for 15 min; and 100 mL of an ethanol aqueous solution (at a volume ratio of 1:1) was added and mixed, then 0.62 g of ethyl orthosilicate (208.33) was added dropwise, the mixture was heated up to 85°C to perform a reaction for 8 h, and the products were washed and dried at the end of the reaction, so that SiO₂@Fe₃O₄ magnetic microspheres were obtained;
(b) preparation of octaglycidyl-polyhedral oligomeric silsesquioxane: 12 g of octavinyl-polyhedral oligomeric silsesquioxane, 8 mL of concentrated sulfuric acid, and 1.5 g of platinum-1,3-divinyl-1,1,3,3-tetramethyldisiloxane were added into 100 mL of toluene and then mixed; the obtained mixture was subjected to heating and refluxing under a nitrogen atmosphere; after that, 30 mL of a trichloromethane solution containing 12 g of allyl glycidyl ether was added dropwise, and heating and refluxing were continued for 8-12 h; and after the reaction was completed, the product was distilled under reduced pressure and then dried, so that octaglycidyl-polyhedral oligomeric silsesquioxane was obtained; and
(c) preparation of carboxyl magnetic beads: 1 g of SiO₂@Fe₃O₄ magnetic microspheres were dispersed in a mixed solution of 2.5 g of octaglycidyl-polyhedral oligomeric silsesquioxane, 0.5 g of a silane coupling agent and 150 mL of water, the dispersion was stirred for a reflux reaction for 3 h, and then the products were subjected to magnetic attraction and washed; the resultant was dispersed in 500 mL of a Tris-HCl buffer with a pH of 8.5, 2 g of dopamine and 0.2 g of stannous octoate were added, and a constant temperature water bath reaction was carried out at 35°C for 6 h; and the products were filtered by magnetic attraction, then 1.2 g of carboxymethyl chitosan (with carboxymethyl substitution degree of 0.90), 6 g of glutaraldehyde, 10 mL of glacial acetic acid and 60 mL of water were added and mixed, and the obtained mixture was enabled to react at 65°C for 4 h while stirring, followed by magnetic attraction, washing and drying, so that carboxyl magnetic beads were obtained.

10 mg of the carboxyl magnetic beads prepared above were added into a 2 mL centrifuge tube, and magnetic separation was carried out to remove supernatant; 400 µL of a 100 mmol/L MES buffer with a pH of 6.0 was added for resuspending the magnetic beads; and 700 µg of a goat anti-rabbit silk fibroin polyclonal antibody was added, 100 µL of a 10 mg/mL coupling activator EDC·HCl solution was then added, and the obtained mixture was placed on an oscillator at room temperature for a coupling reaction for 2 h. After the reaction was completed, the carboxyl magnetic beads were collected by a hopper magnet, and the coupling rate was calculated.

### Embodiment 8

A preparation method of carboxyl magnetic beads includes the following steps:
(a) preparation of SiO₂@Fe₃O₄ magnetic microspheres: 2.47 g of ferric acetylacetonate (353.17) and 7.08 g of 1,12-dodecanediol (202.33, 5) were added into 50 mL of phenyl ether for dissolving, and enabled to react at 190°C for 15 min; and 100 mL of an ethanol aqueous solution (at a volume ratio of 1:1) was added and mixed, then 0.62 g of ethyl orthosilicate (208.33) was added dropwise, the mixture was heated up to 85°C to perform a reaction for 8 h, and the products were washed and dried at the end of the reaction, so that SiO₂@Fe₃O₄ magnetic microspheres were obtained;
(b) preparation of octaglycidyl-polyhedral oligomeric silsesquioxane: 12 g of octavinyl-polyhedral oligomeric silsesquioxane, 8 mL of concentrated sulfuric acid, and 1.5 g of platinum-1,3-divinyl-1,1,3,3-tetramethyldisiloxane were added into 100 mL of toluene and then mixed; the obtained mixture was subjected to heating and refluxing under a nitrogen atmosphere; after that, 30 mL of a trichloromethane solution containing 12 g of allyl glycidyl ether was added dropwise, and heating and refluxing were continued for 8-12 h; and after the reaction was completed, the product was distilled under reduced pressure and then dried, so that octaglycidyl-polyhedral oligomeric silsesquioxane was obtained; and
(c) preparation of carboxyl magnetic beads: 1 g of SiO₂@Fe₃O₄ magnetic microspheres were dispersed in a mixed solution of 2.2 g of octaglycidyl-polyhedral oligomeric silsesquioxane, 0.5 g of a silane coupling agent and 150 mL of water, the dispersion was stirred for a reflux reaction for 3 h, and then the products were subjected to magnetic attraction and washed; the resultant was dispersed in 600 mL of a Tris-HCl buffer with a pH of 8.5, 3.5 g of dopamine and 0.3 g of stannous octoate were added, and a constant temperature water bath reaction was carried out at 35°C for 7 h; and the products were filtered by magnetic attraction, then 1.5 g of carboxymethyl chitosan (with carboxymethyl substitution degree of 0.90), 7 g of glutaraldehyde, 10 mL of glacial acetic acid and 60 mL of water were added and mixed, and the obtained mixture was enabled to react at 60°C for 5 h while stirring, followed by magnetic attraction, washing and drying, so that carboxyl magnetic beads were obtained.

10 mg of the carboxyl magnetic beads prepared above were added into a 2 mL centrifuge tube, and magnetic separation was carried out to remove supernatant; 400 µL of a 100 mmol/L MES buffer with a pH of 6.0 was added for resuspending the magnetic beads; and 700 µg of a goat anti-rabbit silk fibroin polyclonal antibody was added, 100 µL of a 10 mg/mL coupling activator EDC·HCl solution was then added, and the obtained mixture was placed on an oscillator at room temperature for a coupling reaction for 2 h. After the reaction was completed, the carboxyl magnetic beads were collected by a hopper magnet, and the coupling rate was calculated.

### Comparative example 1

The difference from Embodiment 7 was the use of MPS100/carboxyl magnetic beads from Shanghai Yinruicheng Biotechnology Co., Ltd. 10 mg of commercially available carboxyl magnetic beads were added into a 2 mL centrifuge tube, and magnetic separation was carried out to remove supernatant; 400 µL of a 100 mmol/L MES buffer with a pH of 6.0 was added for resuspending the magnetic beads; and 700 µg of a goat anti-rabbit silk fibroin polyclonal antibody was added, 100 µL of a 10 mg/mL coupling activator EDC·HCl solution was then added, and the obtained mixture was placed on an oscillator at room temperature for a coupling reaction for 2 h. After the reaction was completed, the carboxyl magnetic beads were collected by a hopper magnet, and the coupling rate was calculated.

### Comparative example 2

The difference from Embodiment 7 was that the carboxymethyl substitution degree of carboxymethyl chitosan is 1.1.

A preparation method of carboxyl magnetic beads includes the following steps:
(a) preparation of SiO₂@Fe₃O₄ magnetic microspheres: 2.47 g of ferric acetylacetonate (353.17) and 7.08 g of 1,12-dodecanediol (202.33, 5) were added into 50 mL of phenyl ether for dissolving, and enabled to react at 190°C for 15 min; and 100 mL of an ethanol aqueous solution (at a volume ratio of 1:1) was added and mixed, then 0.62 g of ethyl orthosilicate (208.33) was added dropwise, the mixture was heated up to 85°C to perform a reaction for 8 h, and the products were washed and dried at the end of the reaction, so that SiO₂@Fe₃O₄ magnetic microspheres were obtained;
(b) preparation of octaglycidyl-polyhedral oligomeric silsesquioxane: 12 g of octavinyl-polyhedral oligomeric silsesquioxane, 8 mL of concentrated sulfuric acid, and 1.5 g of platinum-1,3-divinyl-1,1,3,3-tetramethyldisiloxane were added into 100 mL of toluene and then mixed; the obtained mixture was subjected to heating and refluxing under a nitrogen atmosphere; after that, 30 mL of a trichloromethane solution containing 12 g of allyl glycidyl ether was added dropwise, and heating and refluxing were continued for 8-12 h; and after the reaction was completed, the product was distilled under reduced pressure and then dried, so that octaglycidyl-polyhedral oligomeric silsesquioxane was obtained; and
(c) preparation of carboxyl magnetic beads: 1 g of SiO₂@Fe₃O₄ magnetic microspheres were dispersed in a mixed solution of 2.5 g of octaglycidyl-polyhedral oligomeric silsesquioxane, 0.5 g of a silane coupling agent and 150 mL of water, the dispersion was stirred for a reflux reaction for 3 h, and then the products were subjected to magnetic attraction and washed; the resultant was dispersed in 500 mL of a Tris-HCl buffer with a pH of 8.5, 2 g of dopamine and 0.2 g of stannous octoate were added, and a constant temperature water bath reaction was carried out at 35°C for 6 h; and the products were filtered by magnetic attraction, then 1.2 g of carboxymethyl chitosan (with carboxymethyl substitution degree of 1.1), 6 g of glutaraldehyde, 10 mL of glacial acetic acid and 60 mL of water were added and mixed, and the obtained mixture was enabled to react at 65°C for 4 h while stirring, followed by magnetic attraction, washing and drying, so that carboxyl magnetic beads were obtained.

10 mg of the carboxyl magnetic beads prepared above were added into a 2 mL centrifuge tube, and magnetic separation was carried out to remove supernatant; 400 µL of a 100 mmol/L MES buffer with a pH of 6.0 was added for resuspending the magnetic beads; and 700 µg of a goat anti-rabbit silk fibroin polyclonal antibody was added, 100 µL of a 10 mg/mL coupling activator EDC·HCl solution was then added, and the obtained mixture was placed on an oscillator at room temperature for a coupling reaction for 2 h. After the reaction was completed, the carboxyl magnetic beads were collected by a hopper magnet, and the coupling rate was calculated.

**Table 1**

| | Embodiment 7 | Embodiment 8 | Comparative example 1 | Comparative example 2 |
|---|---|---|---|---|
| OD value of concentration before coupling | 1.532 | 1.533 | 1.535 | 1.530 |
| OD value of concentration after coupling | 0.428 | 0.444 | 0.537 | 0.490 |
| Coupling rate (%) | 72.06 | 71.04 | 65.02 | 67.97 |

The solution before and after antibody coupling was tested with BCA, the absorbance value was substituted into a standard curve to calculate concentrations, and finally the coupling rate was calculated. Coupling rate (%) = (concentration before coupling-concentration after coupling)/concentration before coupling.

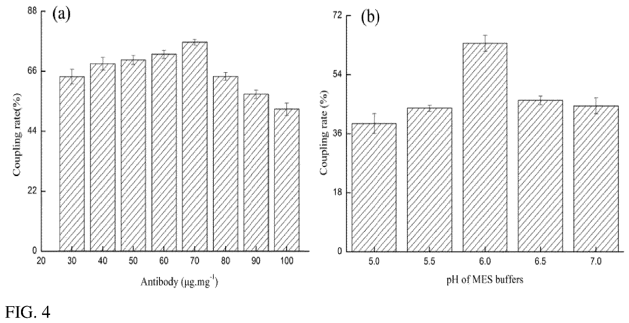

The present invention uses the silk fibroin polyclonal antibody to prepare immunomagnetic beads, and combines the immunomagnetic beads with a chromatography technology to prepare immunomagnetic bead chromatography test paper. FIGs. 1-2 show that the enrichment effect of immunomagnetic beads on silk fibroin is good, and FIG. 3 shows that the test paper can realize rapid and sensitive detection while enriching trace silk fibroin. As shown in FIG. 4, panel (a) shows the result of the effect of different levels of goat anti-rabbit silk fibroin polyclonal antibodies on coupling rates. The results show that when the antibody content is 70 µg/mg, the coupling rate of carboxyl magnetic beads is the highest. Panel (b) shows the results of the effect of MES buffers with different Ph values on coupling rates. The results show that when the Ph value of the MES buffer is 6.0, the coupling rate of carboxyl magnetic beads is the highest.

The carboxyl magnetic beads prepared in the present invention undergo a coupling reaction when the antibody content is 70 µg/mg and the Ph value of the MES buffer is 6.0. As can be seen from Embodiment 7 and Comparative example 1, the carboxyl magnetic beads prepared in the present invention have a higher coupling rate and a better coupling effect compared with the commercially available carboxyl magnetic beads, and thus can achieve more sensitive detection of trace silk fibroin. From Embodiment 7 and Comparative example 2, it can be seen that when the carboxymethyl substitution degree of carboxymethyl chitosan is greater than 1, carboxymethyl substitution occurs simultaneously on the amino groups, which will reduce the reactive functional groups that enable the effective binding between chitosan and magnetic beads, thus being not conducive to increasing the density of carboxy groups on the surfaces of the magnetic beads.

The raw materials and equipment us"d in'the present invention are all common raw materials and equipment in the art unless otherwise specified; and the methods used in the present invention, unless otherwise specified, are conventional methods in the art.

The foregoing descriptions are merely exemplary embodiments of the present invention, and are not intended to limit the present invention in any way. Any simple modifications, changes or equivalent variations made to the above embodiments based on the technical essence of the present invention still fall within the protection scope of the technical solutions of the present invention.

## Claims

1. A preparation method of immunomagnetic bead test strips for rapid detection of trace proteins in cultural relics, **characterized by** comprising the following steps:
(1) enabling carboxyl magnetic beads and a protein antibody to undergo a coupling reaction, carrying out magnetic separation to remove supernatant, and washing with a buffer and a cleaning agent in sequence to obtain immunomagnetic beads;
(2) spraying the immunomagnetic beads onto a pretreated nitrocellulose membrane, and drying for later use; and respectively spraying a diluted antibody solution I and a diluted antibody solution II onto a test line and a control line of the nitrocellulose membrane, and drying for later use; and
(3) sequentially assembling a sample pad, an immunomagnetic bead binding pad, the nitrocellulose membrane and an absorbent pad and then cutting the assembled product to obtain immunomagnetic bead test strips, drying, and sealing for storage.

2. The preparation method of immunomagnetic bead test strips for rapid detection of trace proteins in cultural relics according to claim 1, **characterized in that**
when the trace protein is silk fibroin, the protein antibody is a goat anti-rabbit silk fibroin polyclonal antibody; the antibody solution I is a mouse anti-rabbit silk fibroin polyclonal antibody solution; and the antibody solution II is a goat anti-rabbit antibody solution; and
when the trace protein is collagen, the protein antibody is a goat anti-rabbit type I collagen polyclonal antibody; the antibody solution I is a mouse anti-rabbit type I collagen polyclonal antibody solution; and the antibody solution II is a goat anti-rabbit antibody solution.

3. The preparation method of immunomagnetic bead test strips for rapid detection of trace proteins in cultural relics according to claim 1, **characterized in that** in step (1), the coupling reaction comprises the following steps: adding the carboxyl magnetic beads into an MES buffer for resuspension, then adding the protein antibody and a coupling activator, and performing the coupling reaction at room temperature for 2-4 h.

4. The preparation method of immunomagnetic bead test strips for rapid detection of trace proteins in cultural relics according to claim 3, **characterized in that** in step (1), the sizes of the carboxyl magnetic beads are 100-300 nm; the mass ratio of the carboxyl magnetic beads to the protein antibody is 10 mg:500 µg; the pH of the MES buffer is 5-7; and the coupling activator is an EDC·HCl solution with a concentration of 10 mg/mL.

5. The preparation method of immunomagnetic bead test strips for rapid detection of trace proteins in cultural relics according to claim 1, **characterized in that** in step (1), the buffer is a Tris-HCl solution, with a pH value being 7.4; and the cleaning agent is a PBST solution containing 0.1 percent by mass of Tween-20, with a pH value being 7.4.

6. The preparation method of immunomagnetic bead test strips for rapid detection of trace proteins in cultural relics according to claim 5, **characterized in that** in step (1), the step of washing with a buffer and a cleaning agent in sequence comprises: adding the carboxyl magnetic beads into the buffer, mixing and resuspending the magnetic beads, rotating and mixing for 2-3 h at room temperature, carrying out magnetic separation to remove supernatant, then adding the cleaning agent, and carrying out magnetic separation to remove supernatant.

7. The preparation method of immunomagnetic bead test strips for rapid detection of trace proteins in cultural relics according to claim 1, **characterized in that** in step (2), the pretreatment refers to soaking in the MES buffer and then drying; and the dilution refers to diluting 500-1000 times with a PBS buffer containing 0.5-2.0 percent by mass of sucrose.

8. The preparation method of immunomagnetic bead test strips for rapid detection of trace proteins in cultural relics according to claim 1, **characterized in that** in step (1), a preparation method of the carboxyl magnetic beads comprises the following steps:
(a) preparation of SiO₂@Fe₃O₄ magnetic microspheres: adding ferric acetylacetonate and 1,12-dodecanediol into phenyl ether for dissolving, and enabling same to react at 180-210°C for 5-20 min; and adding an ethanol aqueous solution and mixing, then adding ethyl orthosilicate dropwise, heating up to 80-100°C to perform a reaction for 6-10 h, washing and drying at the end of the reaction, so as to obtain SiO₂@Fe₃O₄ magnetic microspheres;
(b) preparation of octaglycidyl-polyhedral oligomeric silsesquioxane: mixing octavinyl-polyhedral oligomeric silsesquioxane, concentrated sulfuric acid, platinum-1,3-divinyl-1,1,3,3-tetramethyldisiloxane and toluene, heating and refluxing under an inert atmosphere, adding allyl glycidyl ether and trichloromethane dropwise, and continuing heating and refluxing for 8-12 h; and after the reaction is completed, distilling under reduced pressure, followed by drying, so as to obtain octaglycidyl-polyhedral oligomeric silsesquioxane; and
(c) preparation of carboxyl magnetic beads: dispersing SiO₂@Fe₃O₄ magnetic microspheres in a mixed solution of octaglycidyl-polyhedral oligomeric silsesquioxane, a silane coupling agent and water, stirring for a reflux reaction for 2-4 h, then carrying out magnetic attraction, and washing; dispersing the resultant in a Tris-HCl buffer with a pH of 8-9, adding dopamine and stannous octoate, and enabling same to be subjected to a constant temperature water bath reaction at 30-40°C for 6-8 h; and filtering by magnetic attraction, then adding carboxymethyl chitosan, glutaraldehyde, glacial acetic acid and water and mixing, and enabling the obtained mixture to react at 60-70°C for 3-5 h while stirring, followed by magnetic attraction, washing and drying, so as to obtain carboxyl magnetic beads.

9. The preparation method of immunomagnetic bead test strips for rapid detection of trace proteins in cultural relics according to claim 8, **characterized in that**
in step (a), the molar ratio of the ferric acetylacetonate to the ethyl orthosilicate is (5-10):(2-4); in step (b), the amount ratio of the octavinyl-polyhedral oligomeric silsesquioxane, the concentrated sulfuric acid, the platinum-1,3-divinyl-1,1,3,3-tetramethyldisiloxane, and the toluene is (10-20 g):(5-10 mL):(1-3 g):100 mL; and the amount ratio of the octavinyl-polyhedral oligomeric silsesquioxane, the allyl glycidyl ether, and the trichloromethane is (7-12 g):(11-15 g):(20-40 mL); and
in step (c), the mass ratio of the SiO₂@Fe₃O₄ magnetic microspheres, the octaglycidyl-polyhedral oligomeric silsesquioxane, and the dopamine is 1:(2-3):(2-5); the mass ratio of the dopamine, the carboxymethyl chitosan, and the glutaraldehyde is (2-5):(1-2):(5-10); and the carboxymethyl substitution degree of the carboxymethyl chitosan is not greater than 1.

10. Application of the immunomagnetic bead test strips prepared by the preparation method according to any one of claims 1-9, **characterized by** comprising the following steps: dissolving a sample to be tested in a protein solubilization reagent, taking supernatant and dripping same onto a sample pad of the immunomagnetic bead test strip; after chromatography, observing the color development in a test line and a control line on the immunomagnetic bead test strip; and under the premise of the color development in the control line, if there is no color in the test line, it represents negative, and if there is color in the test line, it represents positive.
